# EUROPEAN PATENT APPLICATION

(11) **EP 3 632 546 A1**
(43) Date of publication of application: **08.04.2020**
(21) Application number: 18810101.8
(22) Date of filing: 31.05.2018
(51) Int. Cl.: B01J 29/035, B01J 29/14, B01J 29/46, B01J 29/68, B01J 29/76, C07B 61/00, C07C 1/06

(54) **FISCHER-TROPSCH SYNTHESIS CATALYST STRUCTURE, PRODUCTION METHOD THEREOF, PRODUCTION METHOD OF LIQUID HYDROCARBONS USING SAID CATALYST STRUCTURE, AND HYDROCARBON PRODUCTION DEVICE HAVING SAID CATALYST STRUCTURES**

(30) Priority: 31.05.2017 JP 2017108632
(71) Applicant: Furukawa Electric Co., Ltd., Chiyoda-ku Tokyo 100-8322 (JP)
(72) Inventor: MASUDA, Takao, Hokkaido 0600808 (JP); NAKASAKA, Yuta, Hokkaido 0600808 (JP); YOSHIKAWA, Takuya, Hokkaido 0600808 (JP); KATO, Sadahiro, Tokyo 100-8322 (JP); FUKUSHIMA, Masayuki, Tokyo 100-8322 (JP); TAKAHASHI, Hiroko, Tokyo 100-8322 (JP); BANBA, Yuichiro, Tokyo 100-8322 (JP); SEKINE, Kaori, Tokyo 100-8322 (JP)
(74) Representative: Gunzelmann, Rainer
(86) International application number: PCT/JP2018/021088
(87) International publication number: WO 2018/221700

(57) **Abstract**

A structured catalyst for Fischer-Tropsch synthesis that can prevent aggregation of metal nanoparticles, and suppress a deterioration in catalytic activity to achieve a long life time, and that does not require a complicated replacement operation, and can save resources, a method for manufacturing the same, and a method for manufacturing a liquid hydrocarbon by using the structured catalyst, and a manufacturing apparatus for hydrocarbon using the structured catalyst, are provided. The structured catalyst for Fischer-Tropsch synthesis (1) includes a support (10) of a porous structure including a zeolite-type compound, and one or more metal nanoparticles (20) present in the support (10), the support (10) has channels (11) connecting with each other, and the metal nanoparticles (20) is present at least in the channel (11) of the support (10).

## Description

### Technical Field

The present invention relates to a structured catalyst for Fischer-Tropsch synthesis, a method for manufacturing the same, and a method for manufacturing a liquid hydrocarbon by using the structured catalyst and a manufacturing apparatus for hydrocarbon using the structured catalyst.

### Background Art

As a method for manufacturing a hydrocarbon compound that is used as a source material for a liquid fuel product, such as synthetic oil and synthetic fuel which is alternative fuel for petroleum, a Fischer-Tropsch synthesis reaction (hereinafter, also referred to as a "FT synthesis reaction") for synthesizing hydrocarbon, particularly liquid hydrocarbon, from synthetic gas containing carbon monoxide gas (CO) and hydrogen gas (H₂) as main components by employing a catalytic reaction is known. As a catalyst to be used in the FT synthesis reaction, Patent Document 1, for example, discloses a catalyst including an active metal such as cobalt, or iron supported on a support such as silica, or alumina, and Patent Document 2 discloses a catalyst containing cobalt, zirconium, titanium, and silica.

The catalyst to be used in the FT synthesis reaction can be obtained, for example, by impregnating a support such as silica, or alumina with cobalt oxide and/or ruthenium oxide and calcinating the impregnated support, to produce a catalyst in which cobalt oxide and/or ruthenium oxide is supported (a catalyst before reduction). In order for the catalyst obtained in this manner to exhibit sufficient activity for the FT synthesis reaction, the catalyst needs to be reduced by bringing the catalyst into contact with reducing gas such as hydrogen gas and converting cobalt and/or ruthenium, which serves as an active metal, from an oxide state to a metal state, as disclosed in Patent Document 3.

Incidentally, the FT synthesis reaction accompanies extremely large heat generation as disclosed in Patent Document 4, so it is known that local overheating may occur on a catalyst surface, and thus a side reaction (such as carbonaceous precipitation) may proceed in a hot spot generated on the catalyst surface to deteriorate the activity. In order to prevent the generation of such a hot spot, active points need to be dispersed to prevent aggregation of active metal species (metal nanoparticles) acting as the catalyst. In order to prevent aggregation of the active metal species, it is conceivable to use a support exhibiting a strong interaction with the active metal species such that the metal nanoparticles may not easily aggregate.

As an example of this method, a sol-gel method by which metal nanoparticles are supported in a highly dispersed state is known. In the sol-gel method, the active metal species is uniformly introduced at an atomic level during synthesis of a metal oxide serving as a support. Active metal species of a metal supported catalyst is included in a very highly dispersed state in a lattice of the metal oxide serving as a support, so the active metal species of the metal supported catalyst does not easily aggregate in various processes or reactions. However, due to a strong bond of the active metal species to the support, activation of the catalyst prior to the reaction becomes difficult, and there is a problem that sufficient catalytic activity cannot be achieved.

In addition, when the aggregation of the metal nanoparticles occurs, as an effective surface area of the catalyst decreases, the catalytic activity deteriorates. Thus, a life time of the catalyst itself is shorter than the normal life time. Therefore, the catalyst itself needs to be replaced or regenerated in a short period of time, and there are problems such as complicated replacement operation and little resource savings.

### Citation List

### Patent Literatures

Patent Document 1: JP 4-227847 A
Patent Document 2: JP 59-102440 A
Patent Document 3: WO 2015/072573
Patent Document 4: JP 2000-70720 A

### Summary of Invention

### Technical Problem

An object of the present invention is to provide a structured catalyst for Fischer-Tropsch synthesis that can prevent aggregation of metal nanoparticles, and suppress a deterioration in catalytic activity to achieve a long life time, and that does not require a complicated replacement operation, and can save resources, a method for manufacturing the same, and a method for manufacturing a liquid hydrocarbon by using the structured catalyst and a manufacturing apparatus for hydrocarbon using the structured catalyst.

### Solution to Problem

As a result of diligent research to achieve the object described above, the present inventors have found that a structured catalyst that can prevent aggregation of metal nanoparticles, and suppress a deterioration in catalytic activity to achieve a long life time can be obtained by a structured catalyst for Fischer-Tropsch synthesis including a support having a porous structure composed of a zeolite-type compound and one or more metal nanoparticles present in the support, in which the support includes channels connecting with each other, and the metal nanoparticles are present at least at the channels of the support, and thus have completed the present invention based on the finding.

In other words, the summary configurations of the present invention are as follows.
[1] A structured catalyst for Fischer-Tropsch synthesis including a support of a porous structure including a zeolite-type compound and one or more metal nanoparticles present in the support, in which the support has channels connecting with each other, and the metal nanoparticles is present at least at the channels of the support.
[2] The structured catalyst for Fischer-Tropsch synthesis according to [1] described above, wherein
   the channels have any one of a one-dimensional pore, a two-dimensional pore, and a three-dimensional pore defined by a framework of the zeolite-type compound and an enlarged pore portion different from any of the one-dimensional pore, the two-dimensional pore, and the three-dimensional pore, and
   the metal nanoparticles are present at least in the enlarged pore portion.
[3] The structured catalyst for Fischer-Tropsch synthesis according to [2] described above, wherein
   the enlarged pore portion causes a plurality of pores constituting any one of the one-dimensional pore, the two-dimensional pore, and the three-dimensional pore to connect with each other.
[4] The structured catalyst for Fischer-Tropsch synthesis according to [2] or [3] described above, wherein
   an average particle size of the metal nanoparticles is larger than an average inner diameter of the channels and is smaller than or equal to an inner diameter of the enlarged pore portion.
[5] The structured catalyst for Fischer-Tropsch synthesis according to any one of [1] to [4] described above, wherein
   a metal element (M) of the metal nanoparticles is included in an amount from 0.5 to 2.5 mass% with respect to the structured catalyst for Fischer-Tropsch synthesis.
[6] The structured catalyst for Fischer-Tropsch synthesis according to any one of [1] to [5] described above, wherein
   the metal nanoparticles include Co, Fe, Ni, or an alloy containing at least one type thereof.
[7] The structured catalyst for Fischer-Tropsch synthesis according to any one of [1] to [6] described above, wherein
   the average particle size of the metal nanoparticles is from 0.08 nm to 30 nm.
[8] The structured catalyst for Fischer-Tropsch synthesis according to [7] described above, wherein
   the average particle size of the metal nanoparticles is from 0.4 nm to 11.0 nm.
[9] The structured catalyst for Fischer-Tropsch synthesis according to any one of [1] to [8] described above, wherein
   a ratio of the average particle size of the metal nanoparticles to the average inner diameter of the channels is from 0.05 to 300.
[10] The structured catalyst for Fischer-Tropsch synthesis according to [9] described above, wherein
   the ratio of the average particle size of the metal nanoparticles to the average inner diameter of the channels is from 0.1 to 30.
[11] The structured catalyst for Fischer-Tropsch synthesis according to [10] described above, wherein
   the ratio of the average particle size of the metal nanoparticles to the average inner diameter of the channels is from 1.4 to 3.6.
[12] The structured catalyst for Fischer-Tropsch synthesis according to any one of [1] to [11] described above, wherein
   the channels have any one of a one-dimensional pore, a two-dimensional pore, and a three-dimensional pore defined by the framework of the zeolite-type compound and an enlarged pore portion different from any of the one-dimensional pore, the two-dimensional pore, and the three-dimensional pore,
   the average inner diameter of the channels is from 0.1 nm to 1.5 nm, and
   the inner portion of the enlarged pore portion is from 0.5 nm to 50 nm.
[13] The structured catalyst for Fischer-Tropsch synthesis according to any one of [1] to [12] described above, further including
   one or more other metal nanoparticles held on an outer surface of the support.
[14] The structured catalyst for Fischer-Tropsch synthesis according to [13] described above, wherein
   a content of the one or more metal nanoparticles present in the support is larger than a content of the one or more other metal nanoparticles maintained on the outer surface of the support.
[15] The structured catalyst for Fischer-Tropsch synthesis according to any one of [1] to [14] described above, wherein
   the zeolite-type compound is a silicate compound.
[16] A manufacturing apparatus for hydrocarbon using the structured catalyst for Fischer-Tropsch synthesis according to any one of [1] to [15] described above.
[17] A method for manufacturing a structured catalyst for Fischer-Tropsch synthesis, including
   calcinating a precursor material (B) obtained by impregnating a precursor material (A) for obtaining a support having a porous structure composed of a zeolite-type compound with a metal containing solution;
   performing a hydrothermal treatment on a precursor material (C) obtained by the calcinating of the precursor material (B); and
   reducing the hydrothermal-treated precursor material (C).
[18] The method for manufacturing the structured catalyst for Fischer-Tropsch synthesis according to [17] described above, wherein
   from 50 to 500 mass% of a non-ionic surfactant is added with respect to the precursor material (A) before the calcinating.
[19] The method for manufacturing the structured catalyst for Fischer-Tropsch synthesis according to [17] or [18] described above, wherein
   the impregnating the precursor material (A) with the metal containing solution is performed by adding the metal containing solution to the precursor material (A) in multiple portions before the calcinating.
[20] The method for manufacturing the structured catalyst for Fischer-Tropsch synthesis according to any one of [17] to [19] described above, wherein
   in the impregnating the precursor material (A) with the metal containing solution before the calcinating, an added amount of the metal containing solution added to the precursor material (A) is adjusted to make a converted ratio of silicon (Si) constituting the precursor material (A) to a metal element (M) included in the metal containing solution added to the precursor material (A) (atomic ratio Si/M) to be from 10 to 1000.
[21] The method for manufacturing the structured catalyst for Fischer-Tropsch synthesis according to [17] described above, wherein
   in the performing the hydrothermal treatment, the precursor material (C) and a structure directing agent are mixed.
[22] The method for manufacturing the structured catalyst for Fischer-Tropsch synthesis according to [17] described above, wherein
   the performing the hydrothermal treatment is performed under a basic condition.
[23] A method for manufacturing a liquid hydrocarbon by synthesizing the liquid hydrocarbon from carbon monoxide and hydrogen by using a catalyst, the catalyst including
   a support having a porous structure including a zeolite-type compound, and
   one or more metal nanoparticles present in the support,
   a structured catalyst for Fischer-Tropsch synthesis including the support with channels connecting with each other, and
   the metal nanoparticles being present at least at an enlarged pore portion of the channel of the support.

### Advantageous Effects of Invention

According to the present invention, it is possible to provide a structured catalyst for Fischer-Tropsch synthesis that can prevent aggregation of metal nanoparticles and suppress a deterioration in catalytic activity to achieve a long life time, and that does not require a complicated replacement operation, and can save resources, a method for manufacturing the same, and a method for manufacturing a hydrocarbon by using the structured catalyst, and a manufacturing apparatus for hydrocarbon using the structured catalyst.

### Brief Description of Drawings

FIG. 1A and FIG. 1B schematically illustrate a structured catalyst for Fischer-Tropsch synthesis according to an embodiment of the present invention so that an inner structure thereof can be understood. FIG. 1A is a perspective view (partially illustrated in cross-section), and FIG. 1B is a partially enlarged cross-sectional view.
FIG. 2A and FIG. 2B are partial enlarged cross-sectional views for explaining an example of the structured catalyst for Fischer-Tropsch synthesis in FIG. 1. FIG. 2A is a diagram for explaining a sieve function, and FIG. 2B is a diagram for explaining a catalytic capacity.
FIG. 3 is a flowchart illustrating an example of a method for manufacturing the structured catalyst for Fischer-Tropsch synthesis in FIG. 1.
FIG. 4 is a schematic view illustrating a variation of the structured catalyst for Fischer-Tropsch synthesis in FIG. 1.

### Description of Embodiments

Hereinafter, embodiments of the present invention will be described in detail with reference to the drawings.

### Configuration of Fischer-Tropsch Synthesis Structured Catalyst

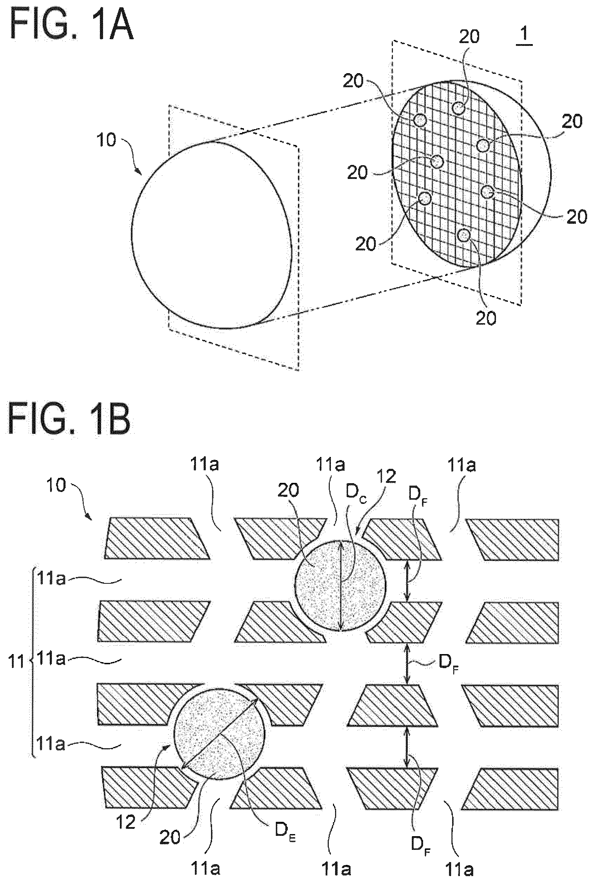

FIG. 1A and FIG. 1B are diagrams schematically illustrating a configuration of a structured catalyst for Fischer-Tropsch synthesis (hereinafter, also referred to simply as the "structured catalyst") according to an embodiment of the present invention. FIG. 1A is a perspective view (partially illustrated in cross-section), and FIG. 1B is a partially enlarged cross-sectional view. Note that the structured catalyst in FIG. 1 is an example thereof, and the shape, dimensions, and the like of each of the configurations according to the present invention are not limited to those illustrated in FIG. 1.

As illustrated in FIG. 1A, a structured catalyst 1 includes a support 10 having a porous structure including a zeolite-type compound, and one or more metal nanoparticles 20 present in the support 10.

In the structured catalyst 1, a plurality of metal nanoparticles 20, 20, ... are included in the porous structure of the support 10. The metal nanoparticles 20 are a catalyst material having catalytic capacity (catalytic activity). The metal nanoparticles will be described in details below. Furthermore, the metal nanoparticles 20 may be a metal oxide, a metal alloy, or a particle containing a composite material thereof.

The support 10 has a porous structure, and as illustrated in FIG. 1B, preferably has a plurality of pores 11a, 11a, ... and channels 11 connecting with each other. Here, the metal nanoparticles 20 are present at least at the channel 11 of the support 10, and are preferably held at least at the channel 11 of the support 10.

With such a configuration, movement of the metal nanoparticles 20, 20 within the support 10 is restricted, and aggregation of the metal nanoparticles 20 is effectively prevented. As a result, a decrease in effective surface area as the metal nanoparticles 20 can be effectively suppressed, and the catalytic activity of the metal nanoparticles 20 lasts for a long period of time. In other words, according to the structured catalyst 1, a deterioration in catalytic activity due to the aggregation of the metal nanoparticles 20 can be suppressed, and a life time of the structured catalyst 1 can be extended. In addition, due to the long life time of the structured catalyst 1, a replacement frequency of the structured catalyst 1 can be reduced, and an amount of waste of the used structured catalyst 1 can be significantly reduced, and thereby resources can be saved.

Typically, when the structured catalyst is used in a fluid (for example, heavy oil, reformed gas such as NOx), it may be subjected to external forces from the fluid. In this case, when the metal nanoparticles are deposited and held only on an outer surface of the support 10, there is a problem that the metal nanoparticles are easily disengaged from the outer surface of the support 10 due to influence of the external force from the fluid. In contrast, in the structured catalyst 1, the metal nanoparticles 20 are held at least in the channel 11 of the support 10, and therefore, even when the metal nanoparticles 20 are subjected to the external force caused by the fluid, the metal nanoparticles 20 are unlikely to be disengaged from the support 10. That is, when the structured catalyst 1 is in the fluid, the fluid flows into the channel 11 from the pore 11a of the support 10, and a speed of the fluid flowing through the channel 11 is slower than a speed of the fluid flowing on the outer surface of the support 10 due to channel resistance (frictional force). Due to influence of such channel resistance, pressure exerted on the metal nanoparticles 20 held at the channel 11 from the fluid is lower than pressure exerted on the metal nanoparticles outside of the support 10 from the fluid. As a result, disengagement of the metal nanoparticles 20 present in the support 10 can be effectively suppressed, and the catalytic activity of the metal nanoparticles 20 can be stably maintained over a long period of time. Note that the channel resistance as described above is thought to be larger as the channels 11 of the support 10 have a plurality of bends and branches, and an interior of the support 10 has a more complex three-dimensional structure.

In addition, the channels 11 preferably have any one of a one-dimensional pore, a two-dimensional pore, and a three-dimensional pore defined by a framework of the zeolite-type compound and an enlarged pore portion 12 different from any of the one-dimensional pore, the two-dimensional pore, and the three-dimensional pore. In this case, the metal nanoparticles 20 are preferably present at least in the enlarged pore portion 12. More preferably, the metal nanoparticles 20 are included at least in the enlarged pore portion 12. Additionally, the enlarged pore portion 12 preferably connects the plurality of pores 11a, 11a constituting any one of the one-dimensional pore, the two-dimensional pore, and the three-dimensional pore with each other. As a result, a separate channel different from the one-dimensional pore, the two-dimensional pore, or the three-dimensional pore is provided in the interior of the support 10, and a function of the metal nanoparticles 20 can be further exhibited. Herein, the "one-dimensional pore" refers to a tunnel-type or cage-type pore forming a one-dimensional channel, or a plurality of tunnel-type or cage-type pores (a plurality of one-dimensional channels) forming a plurality of one-dimensional channels. Also, the "two-dimensional pore" refers to a two-dimensional channel in which a plurality of one-dimensional channels are connected two-dimensionally. The "three-dimensional pore" refers to a three-dimensional channel in which a plurality of one-dimensional channels are connected three-dimensionally. As a result, the movement of the metal nanoparticles 20 within the support 10 is further restricted, and it is possible to further effectively prevent disengagement of the metal nanoparticles 20 and aggregation of the metal nanoparticles 20, 20. The term "include" refers to a state in which the metal nanoparticles 20 are enclosed within the support 10. At this time, the metal nanoparticles 20 and the support 10 need not necessarily be in direct contact with each other, but the metal nanoparticles 20 may be indirectly held by the support 10 with other substances (for example, a surfactant, or the like) interposed between the metal nanoparticles 20 and the support 10.

Although FIG. 1B illustrates a case in which the metal nanoparticles 20 are included in the enlarged pore portion 12, the configuration is not limited to this configuration only, and the metal nanoparticles 20 may be held at the channel 11 with a part of the metal nanoparticles 20 protruding to an outer side of the enlarged pore portion 12. Furthermore, the metal nanoparticles 20 may be partially embedded in a portion of the channel 11 other than the enlarged pore portion 12 (for example, an inner wall portion of the channel 11), or may be held by being fixed.

In addition, the channels 11 are three-dimensionally formed including a branch portion or a merging portion within the support 10, and the enlarged pore portion 12 is preferably provided at the branch portions or the merging portions of the channel 11.

An average inner diameter D_{F} of the channels 11 formed in the support 10 is calculated from the average lengths of the minor axes and the major axes of the pores 11a constituting any one of the one-dimensional pore, the two-dimensional pore, and the three-dimensional pore, and is, for example, from 0.1 nm to 1.5 nm, and preferably from 0.5 nm to 0.8 nm. An inner diameter D_{E} of the enlarged pore portion 12 is from 0.5 nm to 50 nm, for example. The inner diameter D_{E} is preferably from 1.1 nm to 40 nm, and more preferably from 1.1 nm to 3.3 nm. For example, the inner diameter D_{E} of the enlarged pore portion 12 depends on a fine pore diameter of the precursor material (A) described below and an average particle size D_{C} of the metal nanoparticles 20 that are included. The inner diameter D_{E} of the enlarged pore portion 12 has a size which enables inclusion of the metal nanoparticles 20.

The support 10 includes a zeolite-type compound. Examples of the zeolite-type compound include, for example, silicate compounds such as zeolites (aluminosilicate salts), cation exchanged zeolites, and silicalite; zeolite analog compounds such as aluminoborate salts, aluminoarsenate salts, and germanate salts; and phosphate-based zeolite analog materials such as molybdenum phosphates. Among these, the zeolite-type compound is preferably a silicate compound.

A framework of the zeolite-type compound is selected from FAU type (Y type or X type), MTW type, MFI type (ZSM-5), FER type (ferrierite), LTA type (A type), MWW type (MCM-22), MOR type (mordenite), LTL type (L type), and BEA type (beta type). Preferably, it is MFI type, and more preferably ZSM-5. In the zeolite-type compound, a plurality of pores are formed, each of which has a pore diameter corresponding to the respective framework. For example, the maximum pore diameter of the MFI type is 0.636 nm (6.36 Å) and the average pore diameter is 0.560 nm (5.60 Å).

There are cases where the metal nanoparticles 20 are primary particles, and cases where the metal nanoparticles 20 are secondary particles including aggregated primary particles. The average particle size D_{C} of the metal nanoparticles 20 is preferably greater than the average inner diameter D_{F} of the channels 11 and not greater than the inner diameter D_{E} of the enlarged pore portion 12 (D_{F} < D_{C} ≤ D_{E}). Such metal nanoparticles 20 are suitable included in the enlarged pore portion 12 inside the channels 11, to restrict movement of the metal nanoparticles 20 within the support 10. Thus, even when the metal nanoparticles 20 are subjected to the external force from the fluid, the movement of the metal nanoparticles 20 within the support 10 is suppressed to make it possible to effectively prevent the metal nanoparticles 20, 20, ... included within the respective enlarged pore portions 12, 12, ... dispersed at the channels 11 of the support 10 from coming into contact with each other.

In addition, the average particle size D_{C} of the metal nanoparticles 20, for both the primary particles and the secondary particles, is preferably from 0.08 nm to 30 nm, is more preferably equal to or larger than 0.1 nm and less than 25 nm, is further preferably from 0.4 nm to 11.0 nm, and is particularly preferably from 0.8 nm to 2.7 nm. Furthermore, a ratio (D_{C}/D_{F}) of the average particle size D_{C} of the metal nanoparticles 20 with respect to the average inner diameter D_{F} of the channels 11 is preferably from 0.05 to 300, more preferably from 0.1 to 30, even more preferably from 1.1 to 30, and particularly preferably from 1.4 to 3.6. Additionally, the metal element (M) of the metal nanoparticles 20 is preferably included in from 0.5 to 2.5 mass% with respect to the structured catalyst 1, and more preferably from 0.5 to 1.5 mass% for the structured catalyst 1. For example, when the metal element (M) is Co, a content of Co element (mass%) is expressed as {(mass of the Co element)/(mass of all elements of the structured catalyst 1)} × 100.

The metal nanoparticles only may be constituted by a metal that is not oxidized, and may be constituted by a single metal or a mixture of two or more types of metals, for example. Note that in the present specification, the "metal" constituting the metal nanoparticles (as a raw material) refers to an elemental metal containing one type of metal element (M) and a metal alloy containing two or more types of metal elements (M), and the term is a generic term for a metal containing one or more types metal elements (M).

Examples of such a metal include, for example, platinum (Pt), palladium (Pd), ruthenium (Ru), nickel (Ni), cobalt (Co), molybdenum (Mo), tungsten (W), iron (Fe), chromium (Cr), cerium (Ce), copper (Cu), magnesium (Mg), and aluminum (Al), and main components are preferably any one or more types of metals described above, more preferably ruthenium, nickel, iron, and cobalt, even more preferably nickel, iron, and cobalt, and particularly preferably iron and cobalt.

In addition, a ratio (atomic ratio Si/M) of silicon (Si) constituting the support 10 with respect to a metal element (M) constituting the metal nanoparticles 20 is preferably from 10 to 1000, and more preferably from 50 to 200. When the ratio described above is larger than 1000, the action as the catalyst material of the metal nanoparticles may not be sufficiently obtained, as in low activity. On the other hand, when the ratio is smaller than 10, the proportion of the metal nanoparticles 20 becomes too large, and mechanical strength of the support 10 tends to decrease. Note that the metal nanoparticles 20 described herein are nanoparticles that is held within the support 10 or supported by the support 10, and does not include the metal nanoparticles adhering to the outer surface of the support 10.

### Function of Fischer-Tropsch Synthesis Structured Catalyst

The structured catalyst 1 includes the support 10 of a porous structure and one or more metal nanoparticles 20 present in the support, as described above. The structured catalyst 1 exhibits the catalytic activity of the metal nanoparticles 20 by bringing the metal nanoparticles 20 present in the support into contact with a fluid. In particular, the fluid in contact with an outer surface 10a of structured catalyst 1 flows into the interior of the support 10 from the pore 11a formed in the outer surface 10a, and guided into the channels 11, moves through the channels 11, and exits to the exterior of the structured catalyst 1 through another pore 11a. In the pathway through which the fluid moves through the channels 11, contacting with the metal nanoparticles 20 present in the channels 11 results in a catalytic reaction by the metal nanoparticles 20. In addition, the structured catalyst 1 has molecular sieving capability due to the support having a porous structure.

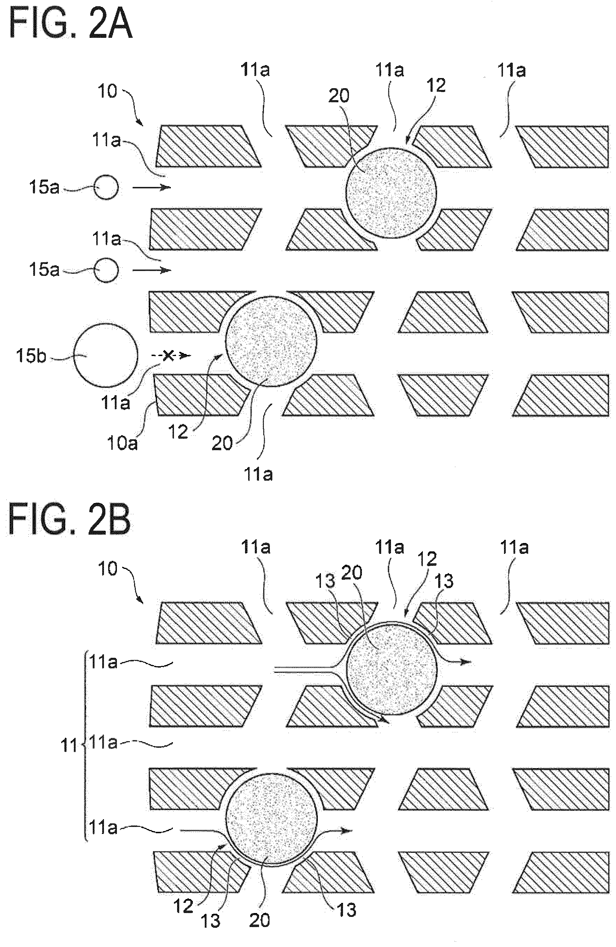

First, the molecular sieving capability of the structured catalyst 1 will be described using FIG. 2A. As illustrated in FIG. 2A, a molecule 15a having a size that is less than or equal to a pore diameter of the pore 11a, in other words, less than or equal to the inner diameter of the channels 11, can enter the support 10. On the other hand, a molecule 15b having a size exceeding the pore diameter of the pore 11a cannot enter the support 10. In this way, when the fluid contains a plurality of types of compounds, the reaction of compounds that cannot enter the support 10 can be restricted and a compound that can enter the support 10 can react.

Of the compounds generated in the support 10 by the reaction, only the compounds composed of molecules having a size less than or equal to the pore diameter of the pore 11a can exit through the pore 11a to the exterior of the support 10, and are obtained as reaction products. On the other hand, the compounds that cannot exit to the exterior of the support 10 from the pore 11a can be released to the exterior of the support 10 when converted into a compound made up of molecules having a size that can exit to the exterior of the support 10. In this way, a specified reaction product can be selectively obtained by using the structured catalyst 1.

In the structured catalyst 1, as illustrated in FIG. 2B, the metal nanoparticles 20 are included in the enlarged pore portions 12 of the channels 11. When the average particle size D_{C} of the metal nanoparticles is larger than the average inner diameter D_{F} of the channels 11 and smaller than the inner diameter D_{E} of the enlarged pore portion 12(D_{F} < D_{C} < D_{E}), a small channel 13 is formed between the metal nanoparticles and the enlarged pore portion 12. Thus, as indicated by an arrow in FIG. 2B, the fluid entering the small channel 13 comes into contact with the metal nanoparticles. Because each metal nanoparticles is included in the enlarged pore portion 12, movement in the support 10 is restricted. Therefore, aggregation of the metal nanoparticles in the support 10 is prevented. As a result, a large surface contact area between the metal nanoparticles and the fluid can be stably maintained.

Specifically, when a molecule entering the channels 11 comes into contact with the metal nanoparticles 20, the molecule (modified material) is modified by the catalytic reaction. In the present invention, by using the structured catalyst 1, hydrocarbon (excluding CH₄), preferably C₅ to C₂₀ hydrocarbons, and particularly liquid hydrocarbons (C₅ to C₁₆ hydrocarbons) that are liquid at room temperature can be manufactured by using, for example, mixed gas whose main components are hydrogen and carbon monoxide as a source material. This catalytic reaction is carried out at an elevated temperature of, for example, 180°C to 350°C, but the metal nanoparticles 20 are present in the support 10 and are therefore less susceptible to heating. In particular, because the metal nanoparticles 20 are present in the enlarged pore portion 12, the movement of the metal nanoparticles 20 in the support 10 is more restricted, and the aggregation of the metal nanoparticles 20 (calcinating) is further suppressed. As a result, the deterioration in catalytic activity is further suppressed, and a longer life time of the structured catalyst 1 can be achieved. In addition, even when the catalytic activity is deteriorated by using the structured catalyst 1 for a long period of time, the activation treatment (reduction treatment) of the metal nanoparticles 20 can be easily performed since the metal nanoparticles 20 is not bonded to the support 10.

### Method for Manufacturing the Fischer-Tropsch Synthesis Structured Catalyst

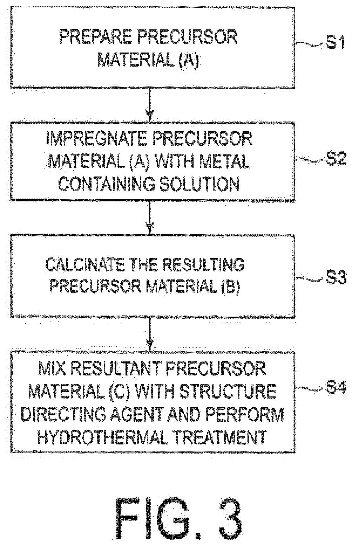

FIG. 3 is a flowchart illustrating a method for manufacturing the structured catalyst 1 in FIG. 1.

### Step S1: Preparation Step

As illustrated in FIG. 3, the precursor material (A) is first prepared for obtaining the support having the porous structure including the zeolite-type compound. The precursor material (A) is preferably a regular mesoporous material, and can be appropriately selected according to the type (composition) of the zeolite-type compound constituting the support of the structured catalyst.

Here, when the zeolite-type compound constituting the support of the structured catalyst is a silicate compound, the regular mesoporous material is preferably a compound including a Si-O skeleton in which fine pores having a fine pore diameter from 1 to 50 nm are uniformly sized and regularly developed one-dimensionally, two-dimensionally, or three-dimensionally. Such a regular mesoporous material is obtained as a variety of synthetic materials depending on the synthetic conditions, and specific examples of the synthetic materials include SBA-1, SBA-15, SBA-16, KIT-6, FSM-16, and MCM-41. Among them, MCM-41 is preferred. Note that a fine pore diameter of SBA-1 is from 10 to 30 nm, a fine pore diameter of SBA-15 is from 6 to 10 nm, a fine pore diameter of SBA-16 is 6 nm, a fine pore diameter of KIT-6 is 9 nm, a fine pore diameter of FSM-16 is from 3 to 5 nm, and a fine pore diameter of MCM-41 is from 1 to 10 nm. Examples of such a regular mesoporous material include mesoporous silica, mesoporous aluminosilicate, and mesoporous metallosilicate.

The precursor material (A) may be a commercially available product or a synthetic product. A publicly known method for synthesizing a regular mesoporous material can be conducted in case of synthesizing the precursor material (A). For example, a mixed solution containing a raw material that contains constituent elements of the precursor material (A) and a molding agent for defining the structure of the precursor material (A) is prepared, and the pH is adjusted as necessary and a hydrothermal treatment (hydrothermal synthesis) is performed. Subsequently, a precipitate (product) obtained by the hydrothermal treatment is recovered (e.g., filtered), washed and dried as necessary, and is further subjected to a calcinating treatment to obtain the precursor material (A) that is a regular mesoporous material in a powdery form. Here, examples of a solvent for the mixed solution that can be used include, for example, water, an organic solvent such as alcohol, or a mixed solvent of these. Further, the raw material, which is selected in accordance with the type of the support, and examples of the raw material include, for example, silica agents such as tetraethoxysilane (TEOS); fumed silica; and quartz sand. In addition, various types of surfactants, block copolymers, and the like can be used as the molding agent, which are preferably selected depending on the type of the synthetic material of the regular mesoporous material. A surfactant such as hexadecyltrimethylammonium bromide is preferred when producing MCM-41, for example. The hydrothermal treatment can be performed under treatment conditions at from 0 to 2000 kPa at from 80 to 800°C for from 5 hours to 240 hours within a sealed container. For example, a calcination treatment can be performed under treatment conditions in air, at from 350 to 850°C for from 2 hours to 30 hours.

### Step S2: Impregnating Step

The prepared precursor material (A) is then impregnated with a metal containing solution to obtain the precursor material (B).

The metal containing solution is a solution containing a metal component (e.g., a metal ion) corresponding to the metal element (M) constituting the metal nanoparticles of structured catalyst, and can be prepared, for example, by dissolving a metal salt containing the metal element (M) in a solvent. Examples of such a metal salt include, for example, chlorides, hydroxides, oxides, sulfates, and nitrates, and in particular, nitrates are preferred. Examples of the solvent that can be used include water, an organic solvent such as alcohol, or a mixed solvent thereof.

The method for impregnating the precursor material (A) with the metal containing solution is not particularly limited; however, for example, the metal containing solution is preferably added in portions in a plurality of times while stirring the precursor material (A) in a powdery form prior to the calcination step described below. In addition, a surfactant is preferably added to the precursor material (A) as an additive before adding the metal containing solution to the precursor material (A) from the viewpoint of facilitating infiltration of the metal containing solution into the pores of the precursor material (A). It is believed that such additives serve to cover the outer surface of the precursor material (A) and inhibit the subsequently added metal containing solution from adhering to the outer surface of the precursor material (A), making it easier for the metal containing solution to enter the pores of the precursor material (A).

Examples of such an additive include, for example, non-ionic surfactants such as polyoxyethylene oleyl ether, polyoxyethylene alkyl ether and polyoxyethylene alkylphenyl ether. These surfactants, which cannot infiltrate into the pores of the precursor material (A) due to their large molecular size, do not adhere to the interior of the pores. Thus, these surfactants may not prevent the infiltration of the metal containing solution into the pores. As the method for adding the non-ionic surfactant, for example, it is preferred to add from 50 to 500 mass% of the non-ionic surfactant to the precursor material (A) prior to the calcination step described below. When an added amount of the non-ionic surfactant to the precursor material (A) is less than 50 mass%, the aforementioned suppressing action does not easily occur, and when more than 500 mass% of the non-ionic surfactant is added to the precursor material (A), the viscosity becomes too high, which is not preferable. Thus, the added amount of the non-ionic surfactant to the precursor material (A) is set to a value within the range described above.

In addition, an added amount of the metal containing solution added to the precursor material (A) is preferably adjusted as appropriate in view of the amount of a metal element (M) contained in the metal containing solution with which the precursor material (A) is impregnated (i.e., the amount of the metal element (M) to be present in the precursor material (B)). For example, prior to the calcination step described below, the added amount of the metal containing solution added to the precursor material (A) is adjusted to make a ratio (atomic ratio Si/M) of silicon (Si) constituting the precursor material (A) relative to a metal element (M) included in the metal containing solution added to the precursor material (A) to be from 10 to 1000, and more preferably from 50 to 200. For example, in a case where a surfactant is added to the precursor material (A) as an additive before adding the metal containing solution to the precursor material (A), the added amount of the metal containing solution added to the precursor material (A) is adjusted to make the atomic ratio Si/M to be from 50 to 200, thus the metal element (M) of the metal nanoparticles is included in from 0.5 to 2.5 mass% of the structured catalyst. In the state of the precursor material (B), an amount of a metal element (M) present within the pores is generally proportional to the added amount of the metal containing solution added to the precursor material (A), provided that the metal concentration of the metal containing solution, the presence or absence of the additive, and other conditions such as temperature, pressure, and the like are the same. The amount of the metal element (M) present in the precursor material (B) also has a proportional relationship with the amount of metal element constituting the metal nanoparticles present in the support of the structured catalyst. Accordingly, the added amount of the metal containing solution added to the precursor material (A) is controlled to the range described above, then the metal containing solution can be sufficiently impregnated into the fine pores of the precursor material (A), and, by extension, the amount of metal nanoparticles to be present in the support of the structured catalyst can be adjusted.

After impregnating the precursor material (A) with the metal containing solution, washing treatment may be performed as necessary. Examples of the solvent of the washing solution that can be used include water, an organic solvent such as alcohol, or a mixed solvent thereof. Furthermore, after the precursor material (A) is impregnated with the metal containing solution and the washing treatment is performed as necessary, it is preferred that a drying treatment is further performed. Examples of the drying treatment include overnight air drying and high temperature drying at 150°C or lower. Note that when the calcination treatment described below is performed in a state where the moisture included in the metal containing solution and water in the washing solution is still present in the precursor material (A) in a large amount, the skeletal structure of the precursor material (A) serving as the regular mesoporous material may break down, and thus it is preferred to perform the drying treatment thoroughly.

### Step S3: Calcination Step

Next, the precursor material (B), which is the precursor material (A) for forming the support of porous structure including a zeolite-type compound is impregnated with the metal containing solution, is subjected to a calcination treatment to obtain the precursor material (C).

For example, the calcination treatment is preferably performed under treatment conditions in air, at from 350 to 850°C for 2 hours to 30 hours. With such a calcination treatment, the metal component impregnated into the regular mesoporous material undergoes crystal growth to form a metal nanoparticle within the pore.

### Step S4: Hydrothermal Treatment Step

Next, a mixed solution of the precursor material (C) and the structure directing agent is prepared, and the precursor material (C) obtained by calcinating the precursor material (B) is subjected to hydrothermal treatment to obtain a structured catalyst.

The structure directing agent is a molding agent for defining a skeletal structure of the support of the structured catalyst, where, for example, a surfactant can be used. The structure directing agent is preferably selected according to the skeletal structure of the support of the structured catalyst, where, for example, a surfactant such as tetramethylammonium bromide (TMABr), tetraethylammonium bromide (TEABr), or tetrapropylammonium bromide (TPABr) is preferably used.

The mixing of the precursor material (C) and the structure directing agent may be performed during the hydrothermal treatment step or may be performed before the hydrothermal treatment step. Furthermore, the method for preparing the mixed solution is not particularly limited, where the precursor material (C), the structure directing agent, and a solvent may be mixed simultaneously, or the precursor material (C) and the structure directing agent are each dispersed in a solvent in the solution independently, then the dispersion solutions may be mixed with each other. Examples of the solvent that can be used include, for example, water, an organic solvent such as alcohol, or a mixed solvent of these. In addition, the pH of the mixed solution is preferably adjusted using an acid or a base before performing the hydrothermal treatment.

The hydrothermal treatment can be performed by a publicly known method, and, for example, the hydrothermal treatment can be preferably performed under a treatment condition at from 0 to 2000 kPa at from 80 to 800°C for from 5 hours to 240 hours within a sealed container. Furthermore, the hydrothermal treatment is preferably performed under a basic condition. Although the reaction mechanism here is not necessarily apparent, a hydrothermal treatment is performed using the precursor material (C) as a raw material to cause the skeletal structure of the precursor material (C) as a regular mesoporous material becomes increasingly disintegrated. However, due to an action of the structure directing agent, a new skeletal structure (porous structure) is formed as a support of the structured catalyst, while the positions of the metal nanoparticles are maintained within the pores of the precursor material (C). The structured catalyst obtained in this way includes the support of the porous structure and the metal nanoparticles present in the support, and the support further has the channels in which a plurality of pores connect with each other by the porous structure, and at least part of the metal nanoparticles is present in the channel of the support. In addition, in the hydrothermal treatment step in the present embodiment, a mixed solution in which the precursor material (C) and the structure directing agent are mixed is prepared, and then the precursor material (C) is subjected to the hydrothermal treatment. However, the hydrothermal treatment step is not limited thereto, and the precursor material (C) may be subjected to the hydrothermal treatment without the precursor material (C) being mixed with the structure directing agent.

The precipitate obtained after the hydrothermal treatment (structured catalyst) is preferably washed, dried, and sintered as necessary after recovery (for example, filtration). Examples of the washing solution that can be used include water, an organic solvent such as alcohol, or a mixed solution thereof. Examples of the drying treatment include overnight air drying or high temperature drying at a temperature equal to or lower than 150°C. Note that when the calcination treatment is performed in a state in which there is a large amount of moisture remaining in the precipitate, the skeletal structure as the support of the structured catalyst may disintegrate, and thus it is preferable to perform the drying treatment thoroughly. For example, the calcination treatment can also be performed under treatment conditions in air, at from 350 to 850°C for from 2 hours to 30 hours. Such calcination treatment burns out the structure directing agent adhering to the structured catalyst. Furthermore, the structured catalyst can be used as-is without subjecting the recovered precipitate to a calcination treatment, depending on the intended use. For example, when an environment in which the structured catalyst is used is a high temperature environment of an oxidizing atmosphere, exposing the structured catalyst to the usage environment for a certain period of time allows the structure directing agent to be burned out and to obtain a structured catalyst similar to the structured catalyst that is subjected to calcination treatment. Thus, the obtained structured catalyst can be used as-is.

The manufacturing method described above is an example in which the metal element (M) included in the metal containing solution with which the precursor material (A) is impregnated is a metal species (for example, a precious metal) that is not oxidized easily.

When the metal element (M) included in the metal containing solution, with which the precursor material (A) is impregnated, is a metal species that is easily oxidized (for example, Fe, Co, Cu, and the like), it is preferable to perform reduction treatment on the hydrothermal-treated precursor material (C) after the hydrothermal treatment step described above. When the metal element (M) included in the metal containing solution is a metal species that is easily oxidized, the metal component is oxidized by heat treatment in the steps (steps S3 to S4) after the impregnating treatment step (step S2). Then, metal oxide nanoparticles may be present in the support formed in the hydrothermal treatment step (step S4). Therefore, in order to obtain the structured catalyst in which the metal nanoparticles are present in the support, it is desirable to subject the recovered precipitate after the hydrothermal treatment described above to a calcination treatment, and then to perform reduction treatment in a reducing gas atmosphere such as hydrogen gas. In this case, by performing the reduction treatment, the metal oxide nanoparticles present in the support are reduced, and the metal nanoparticles corresponding to the metal element (M) constituting the metal oxide nanoparticles are formed. As a result, the structured catalyst in which the metal nanoparticles are present in the support is obtained. Note that such reduction treatment may be performed as necessary, for example, when the environment in which the structured catalyst is used is a reducing atmosphere, exposing the metal oxide nanoparticles to a usage environment for a certain period of time allows the metal oxide nanoparticles to be reduced and to form a structured catalyst similar to that when subjected to reduction treatment. Thus, the obtained structured catalyst in which the metal oxide nanoparticles are present in the support can be used as-is.

### Variation of Fischer-Tropsch Synthesis Structured Catalyst

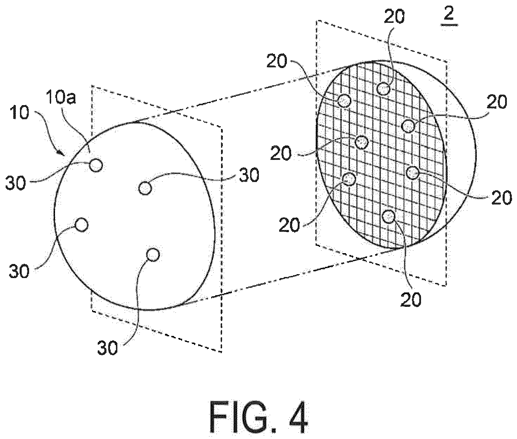

FIG. 4 is a schematic view illustrating a variation of the structured catalyst 1 in FIG. 1. The structured catalyst 1 in FIG. 1 illustrates a case of the structured catalyst 1 including the support 10 and the metal nanoparticles 20 present in the support 10, however, the structured catalyst is not solely limited to this configuration, and, for example, as illustrated in FIG. 4, the structured catalyst 2 may further include one or more other metal nanoparticles 30 held on an outer surface 10a of the support 10.

This metal nanoparticles 30 are catalyst material that exhibits one or a plurality of catalytic activities. A catalytic capacity of the other metal nanoparticles 30 may be the same as or different from the catalytic capacity of the metal nanoparticles 20. Also, when both of the metal nanoparticles 20 and 30 are catalyst materials having the same catalytic activity, a material of the other metal nanoparticles 30 may be the same as or different from the material of the metal nanoparticles 20. According to this configuration, the content of metal nanoparticles held in the structured catalyst 2 can be increased, and the catalytic activity of the metal nanoparticles can be further accelerated.

In this case, the content of the metal nanoparticles 20 present in the support 10 is preferably larger than that of the other metal nanoparticles 30 held on the outer surface 10a of the support 10. This allows the catalytic activity of the metal nanoparticles 20 held within the support 10 to be dominant, to thus exhibit the catalytic activity in a stable manner.

In addition, in the present invention, a method for manufacturing a liquid hydrocarbon by synthesizing the liquid hydrocarbon from carbon monoxide and hydrogen by using a catalyst is provided. The catalyst includes a structured catalyst for Fischer-Tropsch synthesis 1 including the support 10 of the porous structure including the zeolite-type compound, and one or more metal nanoparticles 20 present in the support 10, in which the support 10 includes the channels 11 connecting with each other, and the metal nanoparticles 20 are present at least at the enlarged pore portion 12 of the channel 11 in the support 10. That is, the present invention provides the method for manufacturing the liquid hydrocarbon by synthesizing the liquid hydrocarbon from carbon monoxide and hydrogen by using the structured catalyst for Fischer-Tropsch synthesis described above.

The source material to be used in the method for manufacturing the liquid hydrocarbon employing such a Fischer-Tropsch synthesis reaction is not particularly limited as long as it is a synthetic gas having molecular hydrogen and carbon monoxide as main components, but a synthetic gas having a hydrogen/carbon monoxide molar ratio from 1.5 to 2.5 is suitable, and a synthetic gas having a hydrogen/carbon monoxide molar ratio from 1.8 to 2.2 is more preferable. In addition, reaction conditions of the FT synthesis reaction are not particularly limited, and can be performed under known conditions. For example, a reaction temperature is preferably from 200 to 500°C, from 200 to 350°C, and a pressure is preferably from 0.1 to 3.0 MPa (absolute pressure).

The Fischer-Tropsch synthesis reaction can be carried out in a process known as a reaction process of Fischer-Tropsch synthesis, such as a fixed bed, a supercritical fixed bed, a slurry bed, and a fluidized bed. Preferred processes may include a fixed bed, a supercritical fixed bed, and a slurry bed.

Furthermore, in the present invention, a manufacturing apparatus for hydrocarbon using the structured catalyst described above may be provided. Such a manufacturing apparatus for hydrocarbon is not particularly limited as long as the structured catalyst described above can be used to perform the Fischer-Tropsch synthesis. For example, a commonly used manufacturing apparatus such as a FT synthesis reaction apparatus, and a FT synthesis reaction column can be used. By using the structured catalyst according to the present invention for such a manufacturing apparatus for hydrocarbon, the manufacturing apparatus can achieve the same effects as described above.

Hereinbefore, the structured catalyst, the method for manufacturing the same, and the method for manufacturing the hydrocarbon by using the structured catalyst and the manufacturing apparatus for hydrocarbon using the structured catalyst according to the present embodiment have been described, but the present invention is not limited to the above embodiment, and various modifications and changes are possible based on the technical concept of the present invention.

### Examples

### Examples 1 to 298

### Synthesis of Precursor Material (A)

A mixed aqueous solution was prepared by mixing a silica agent (tetraethoxysilane (TEOS), available from Wako Pure Chemical Industries, Ltd.) and a surfactant as the molding agent. The pH of the mixed aqueous solution was adjusted as appropriate, and hydrothermal treatment was performed at 80 to 350°C for 100 hours in a sealed container. Thereafter, the produced precipitate was filtered out, washed with water and ethanol, and then sintered in the air at 600°C for 24 hours to obtain the precursor material (A) whose type and pore diameter are shown in Tables 1 to 6. Note that the following surfactant was used depending on the type of the precursor material (A) ("the type of the precursor material (A): surfactant").
- MCM-41: hexadecyl trimethyl ammonium bromide (CTAB) (available from Wako Pure Chemical Industries, Ltd.)
- SBA-1: Pluronic P123 (available from BASF SE)

### Preparation of Precursor Materials (B) and (C)

Next, a metal containing aqueous solution was prepared by dissolving a metal salt containing the metal element (M) in water according to the metal element (M) constituting the metal nanoparticles whose type is shown in each of Tables 1 to 6. Note that the metal salt was used according to the type of metal nanoparticles ("metal nanoparticles: metal salt").
- Co: cobalt nitrate (II) hexahydrate (available from Wako Pure Chemical Industries, Ltd.)
- Ni: nickel nitrate (II) hexahydrate (available from Wako Pure Chemical Industries, Ltd.)
- Fe: Iron nitrate (III) nonahydrate (available from Wako Pure Chemical Industries, Ltd.)

Next, the metal containing solution was added to the powdered precursor material (A) in a small amount in a plurality of times, and dried at room temperature (20°C ± 10°C) for 12 hours or longer to obtain the precursor material (B).

Note that when presence or absence of an additive shown in Tables 1 to 6 is "Yes", pretreatment was performed in which an aqueous solution of polyoxyethylene (15) oleyl ether (NIKKOL BO-15 V, available from Nikko Chemicals Co., Ltd.) was added as the additive to the precursor material (A) prior to addition of the metal containing aqueous solution, and then the metal containing aqueous solution was added as described above. Note that when in the presence or absence of an additive is "None", pretreatment with an additive as described above was not performed.

In addition, an added amount of the metal containing aqueous solution added to the precursor material (A) was adjusted to make the ratio (atomic ratio Si/M) of silicon (Si) constituting the precursor material (A) with respect to a metal element (M) included in the metal containing aqueous solution to be the value in Tables 1 to 6.

Next, the precursor material (B) impregnated with the metal containing aqueous solution obtained as described above was subjected to a calcination treatment in air at 600°C for 24 hours to obtain the precursor material (C).

The precursor material (C) obtained as described above and the structure directing agent listed in Tables 1 to 6 were mixed to produce a mixed aqueous solution. Then, a hydrothermal treatment was performed, in a sealed container, under condition of from 80 to 350°C, and at pH and time listed in Tables 1 to 6. Thereafter, the produced precipitate was filtered off, washed with water, dried at 100°C for 12 hours or longer, and was further subjected to calcination treatment in the air at 600°C for 24 hours. The sintered product was then recovered and reduction treatment was performed under inflow of hydrogen gas at 500°C for 60 minutes to obtain the structured catalyst including the support and the metal nanoparticles as the catalyst material shown in Tables 1 to 6 (Examples 1 to 298).

### Comparative Example 1

In Comparative Example 1, cobalt oxide powder (II, III) having an average particle size equal to or less than 50 nm (available from Sigma-Aldrich Japan LLC) was mixed with MFI type silicalite, and similarly to the examples, hydrogen reduction treatment was performed to obtain the structured catalyst in which cobalt nanoparticles as the catalyst material were adhered to an outer surface of the silicalite as the support. The MFI type silicalite was synthesized in the similar manner as in Examples 56 to 62 except for the step of adding a metal.

### Comparative Example 2

In Comparative Example 2, the MFI type silicalite was synthesized in the similar manner as in Comparative Example 1 except that the step of adhering the cobalt nanoparticles was omitted.

### Evaluation

Various characteristic evaluations were performed on the catalyst structural bodies of the above examples and the silicalites of the comparative examples under conditions to be described below.

### [A] Cross-sectional Observation

For each of the catalyst structural bodies of the examples described above and the silicalites of the comparative examples, an observation sample was prepared by using a pulverization method and a cross-sectional observation was conducted using transmission electron microscopy (TEM) (TITAN G2, available from FEI). As a result, it was confirmed that, in each of the catalyst structural bodies of the examples described above, the catalyst material was present and held within the support including silicalite or zeolite. On the other hand, in a case of the silicalite of Comparative Example 1, the catalyst material merely adhered to the outer surface of the support and was not present within the support.

In addition, for the structured catalyst in which the metal was iron nanoparticles (Fe) in the examples described above, a cross-section was fabricated by focused ion beam (FIB) processing, and a section elemental analysis was performed using SEM (SU8020, available from Hitachi High-Technologies Corporation), and EDX (X-Max, available from Horiba, Ltd.). As a result, the element Fe was detected from inside the support. It was confirmed that iron nanoparticles were present within the support from a result of the cross-sectional observation using TEM and SEM/EDX.

### [B] Average Inner Diameter of Channels of Support and Average Particle Size of Catalyst Material

In the TEM image taken by the cross-section observation conducted in the evaluation [A], 500 channels in the support were randomly selected, and the respective major axis length and minor axis length were measured. The respective inner diameters were calculated from the average values (N = 500) of the measured lengths, and the average value of the inner diameters was further determined to be the average inner diameter D_{F} of the channels of the support. In addition, for the catalyst material as well, 500 catalyst materials were randomly selected from the TEM image, and the respective particle sizes were measured (N = 500), and the average value of these was determined to be the average particle size D_{C} of the catalyst materials. The results are shown in Tables 1 to 6.

In addition, SAXS (small angle X-ray scattering) analysis was conducted to confirm the average particle size and dispersion of the catalyst materials. Measurements by the SAXS were performed using a Spring-8 beam line BL19B2. The resultant SAXS data was fitted with a spherical model using the Guinier approximation method, and the particle size was calculated. The particle size was measured for the catalyst structural bodies of which metal is iron nanoparticles. Furthermore, as a comparative reference, a commercially available iron nanoparticles (available from Wako) were observed and measured by under the SEM.

As a result, in commercial products, various sizes of iron nanoparticles were randomly present in a range of particle sizes approximately from 50 nm to 400 nm, whereas in the measurement results of SAXS, scattering peaks with particle sizes of 10 nm or less were also detected in the catalyst structural bodies of each example having an average particle size from 1.2 nm to 2.0 nm determined from the TEM image. From the measurement results of SAXS and the cross-section measurement by SEM/EDX, it was found that catalyst materials having a particle size of 10 nm or less were present within the support with a uniform particle size and in an extremely highly dispersed state.

### [C] Relationship between Added Amount of Metal Containing Solution and Amount of Metal Included in Interior of Support

As shown in Tables 1, 2, 5, and 6, for the structured catalyst in which the metal nanoparticles were cobalt nanoparticles (Co) or iron nanoparticles (Fe) as the catalyst materials, the structured catalyst in which these metal nanoparticles were included within the support was produced with an added amount corresponding to an atomic ratio Si/M of 50, 100, 200, or 1000 (M is Co, Fe). As shown in Tables 3, and 4, for the structured catalyst in which the metal nanoparticles were nickel nanoparticles (Ni) as the catalyst material, the structured catalyst in which the metal nanoparticles were included within the support was produced with an added amount corresponding to an atomic ratio Si/M of 100, 200, or 1000 (M is Ni). Thereafter, the amount of the metal (mass%) that was included within the support of each of the catalyst structural bodies produced with the added amount above was measured. Note that in the present measurement, the structured catalyst having the atomic ratio of Si/M of 100, 200, or 1000 was produced by adjusting the added amount of the metal containing solution in the same manner as the structured catalyst having the atomic ratio Si/M of 100, 200, or 1000 of the Examples 1 to 298, and the structured catalyst having the atomic ratio Si/M of 50 was produced in the same manner as the structured catalyst having the atomic ratio Si/M of 100, 200, or 1000, except that the added amount of the metal containing solution was modified.

The amount of the metal was quantified by radio frequency inductively coupled plasma (ICP) alone or in combination with ICP and X-ray fluorescence analysis (XRF). XRF (energy dispersive fluorescent x-ray analyzer "SEA1200VX", available from SSI Nano Technology Inc.) was performed under conditions of a vacuum atmosphere, and an acceleration voltage 15 kV (by using a Cr filter), or an acceleration voltage 50 kV (by using a Pb filter). XRF is a method for determining the amount of the metal in terms of fluorescence intensity, and XRF alone cannot determine the amount quantitatively (in terms of mass%). Accordingly, an amount of metal of the structured catalyst in which a metal had been added at Si/M = 100 was determined by ICP analysis, while an amount of metal of the structured catalyst in which a metal had been added at Si/M = 50 and less than 100 was calculated based on measurement results of the XRF and measurement results of the ICP.

As a result, it was confirmed that the amount of the metal included in the structured catalyst increased as the added amount of the metal containing solution was increased, at least in a range of the atomic ratio Si/M from 50 to 1000.

### [D] Performance Evaluation

The catalytic activity of the catalyst material was evaluated for the catalyst structural bodies of the examples described above and the silicalites of comparative examples. The results are shown in Tables 1 to 6.

### (1) Catalytic Activity

The catalytic activity was evaluated under the following conditions.

First, 70 mg of the structured catalyst was filled in an atmospheric pressure flow reactor, and hydrogen (8 ml/minute) and carbon monoxide (4 ml/minute) were supplied and heated for 1 hour at 100 to 700°C, under 0.1 MPa to perform the Fischer-Tropsch synthesis. The Single µ-Reactor (Frontier Laboratories Ltd., Rx-3050SR) was used as the atmospheric pressure flow reactor.

After completion of the reaction, the generated gas and the generated liquid that were collected were analyzed by gas chromatography (GC) and gas chromatography mass spectrometry (GC/MS) for the composition. Note that, as the analysis device, TRACE 1310 GC (available from Thermo Fisher Scientific Inc., detector: thermal conductivity detector, flame ionization detector),

And TRACE DSQ (Thermo Fischer Scientific Inc., detector: thermal conductivity detector) were used.

Furthermore, based on the result of the component analysis described above, the product obtained by the FT synthesis reaction was confirmed. In this evaluation, the catalyst structural bodies obtained in the Examples and the Comparative Examples were used to perform the above operations at each of 250°C, 300°C, 350°C, and 400°C, and catalyst activities were determined according to the following evaluation criteria.

When production of hydrocarbons (except for CH₄, the same applies hereinafter) was confirmed at 250°C (that is, when the reaction start temperature was lower than or equal to 250°C), the catalytic activity in the FT synthesis reaction was rated excellent and was denoted as "A". When the production of the hydrocarbons was confirmed at 300°C (in other words, when the reaction start temperature was higher than 250°C and lower than or equal to 300°C), the catalytic activity was rated good and was denoted as "B". When the production of the hydrocarbons was confirmed at 350°C (that is, when the reaction start temperature was higher than 300°C and lower than or equal to 350°C) was rated as a passing level and was denoted as "C" (acceptable) even though the catalytic activity was not good. When the production of the hydrocarbons was confirmed at 400°C (in other words, when the reaction start temperature was higher than 350°C and lower than or equal to 400°C) or the FT synthesis reaction did not occur, the catalytic activity was rated poor and denoted as "D" (unacceptable).

### (2) Durability (Life Time)

The durability was evaluated under the following conditions.

First, the structured catalyst used in the evaluation (1) described above was recovered and heated at 650°C for 12 hours to produce the structured catalyst after heating. Next, the FT synthesis reaction was performed by the similar method as in the evaluation (1) described above using the obtained structured catalyst after heating, and the component analysis of the generated gas and the generated liquid was performed by the similar method as in the evaluation (1) described above.

Based on the obtained result of the analysis, the produced amount of hydrocarbons was determined by the similar method as in the evaluation (1) described above. Furthermore, a degree of achieving a yield of the hydrocarbons with the structured catalyst after the heating was compared to the produced amount of the hydrocarbons with the structured catalyst prior to the heating. Specifically, a percentage (%) of the produced amount of the hydrocarbons with the structured catalyst after the heating (the produced amount determined by the present evaluation (2)) with respect to the produced amount of the hydrocarbons with the structured catalyst prior to the heating (the produced amount determined by the evaluation (1) described above) was calculated.

In the examples, when the produced amount of the hydrocarbons (the produced amount determined by the present evaluation (2)) with the structured catalyst after the heating was more than or equal to 80%, compared to the produced amount of the hydrocarbons with the structured catalyst prior to the heating (the produced amount determined by the evaluation (1) described above), it was rated that durability (heat resistance) was excellent and was denoted as "A". When the produced amount was more than or equal to 60% and less than or equal to 80%, it was rated that the durability (the heat resistance) was good and was denoted as "B". When the produced amount was more than or equal to 40% and less than or equal to 60%, it was rated that the durability (the heat resistance) was not good, but a passing level (acceptable) and denoted as "C". When the produced amount was less than 40%, it was rated that the durability (the heat resistance) was poor (unacceptable) and was denoted as "D".

Performance evaluations similar to the evaluations (1) and (2) described above were also performed on Comparative Examples 1 and 2. Note that Comparative Example 2 was the support itself, and did not include the catalyst material. Therefore, in the performance evaluation described above, only the support of Comparative Example 2 was filled in place of the structured catalyst. The results are shown in Table 6.

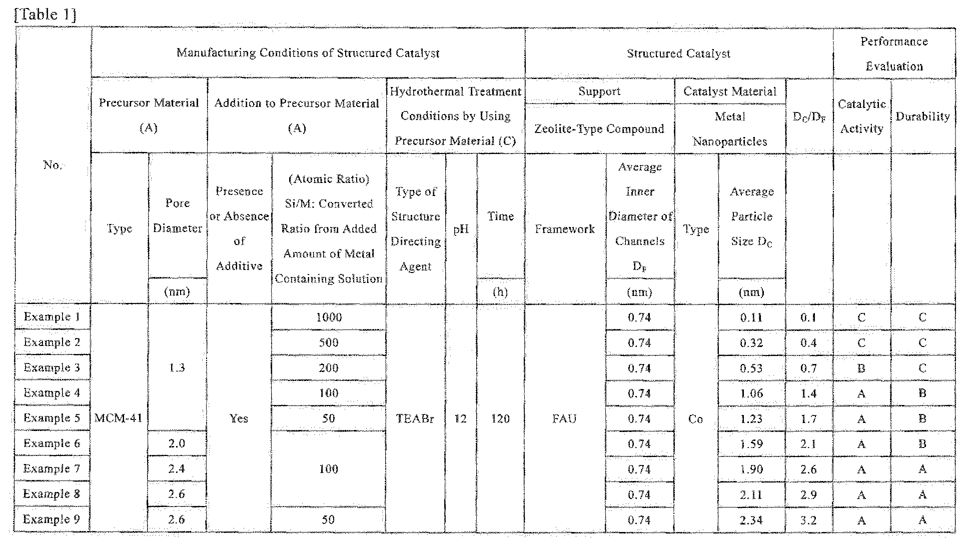

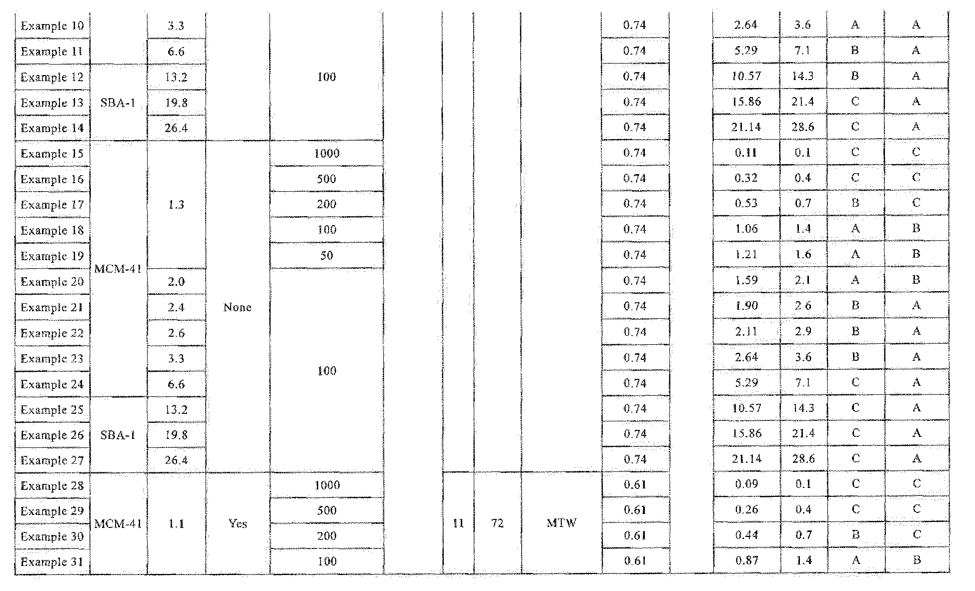

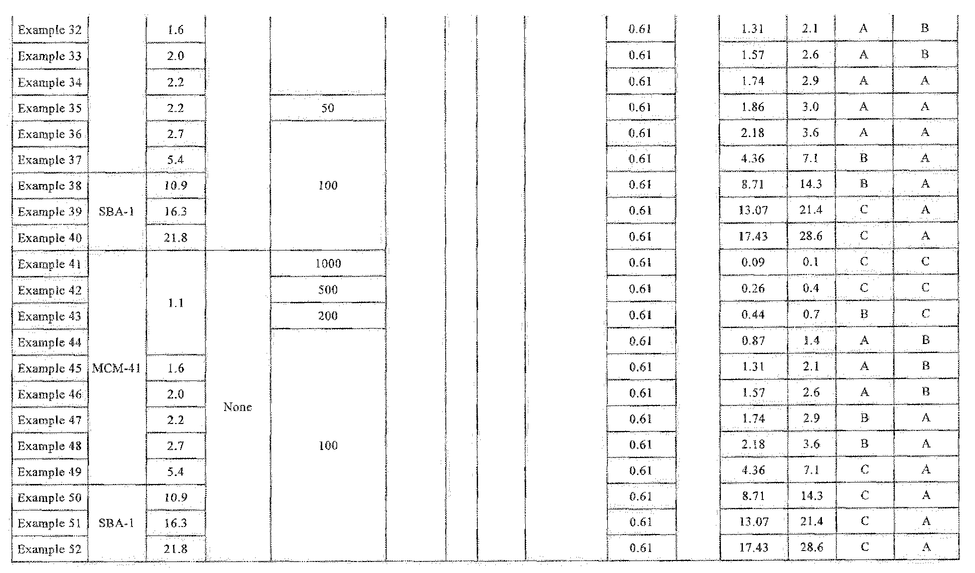

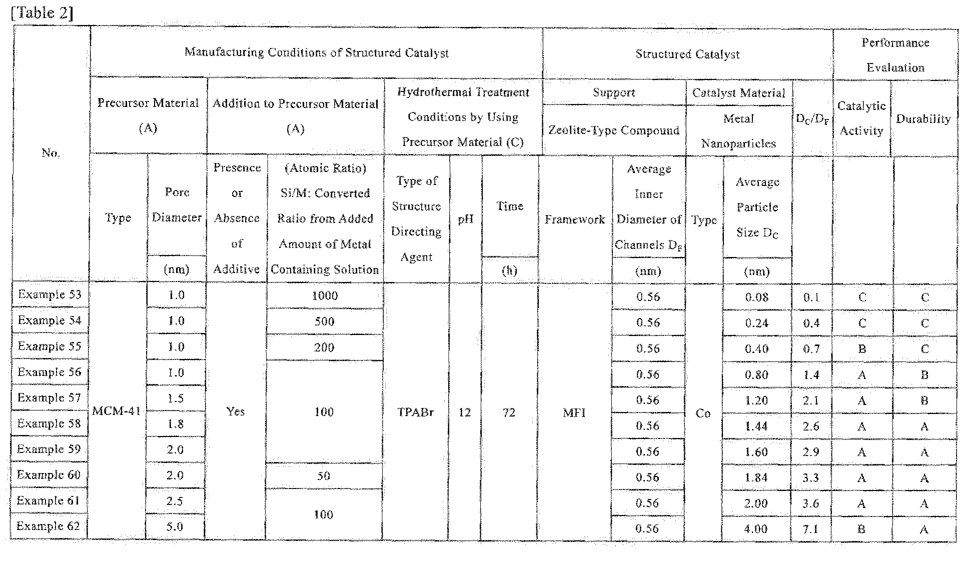

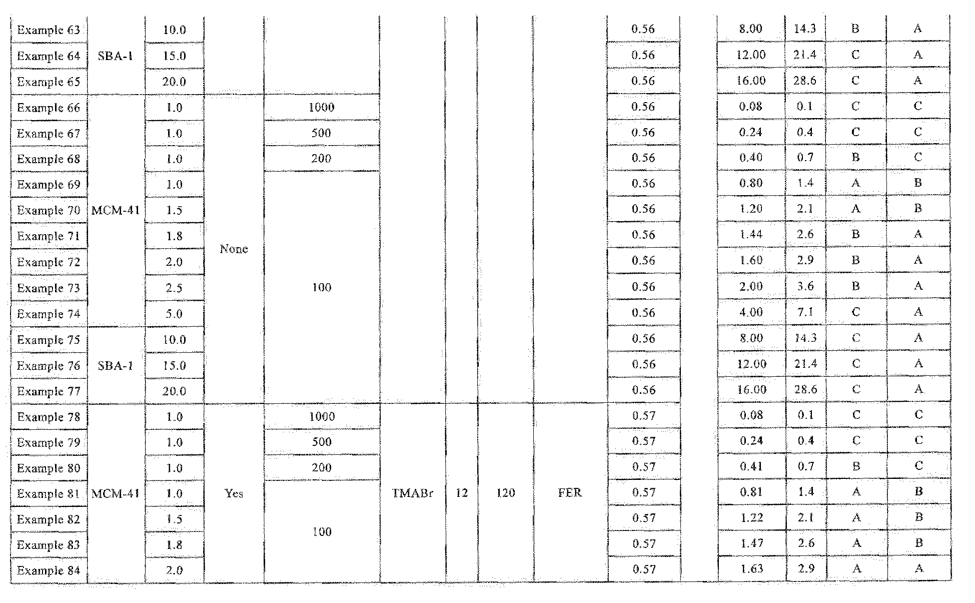

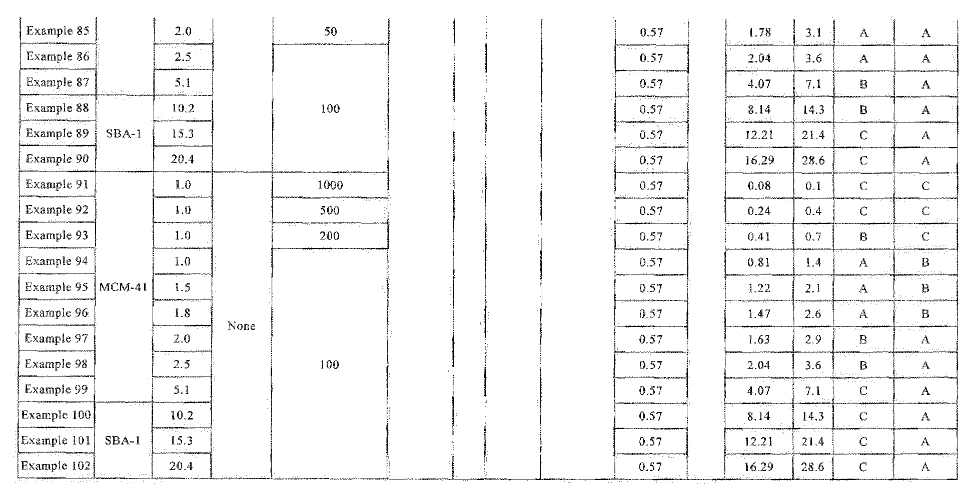

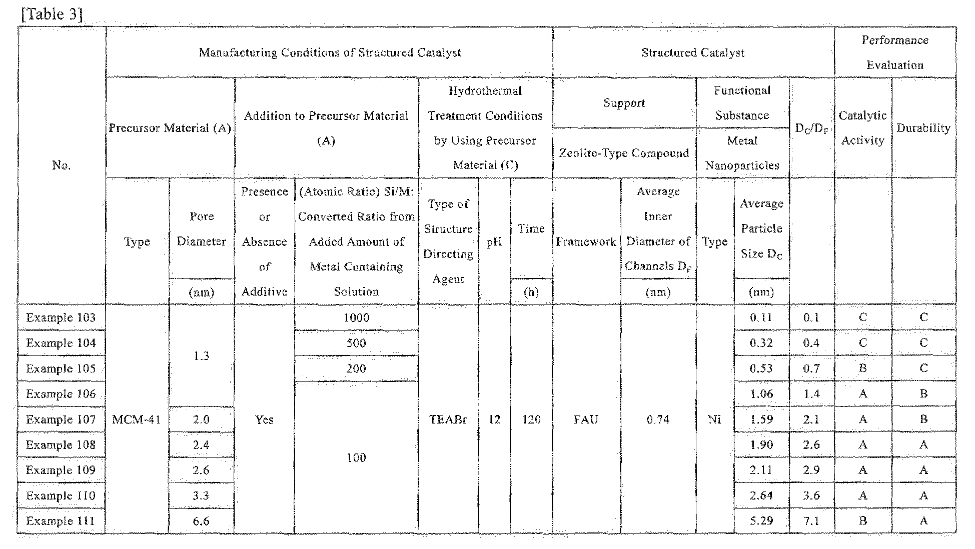

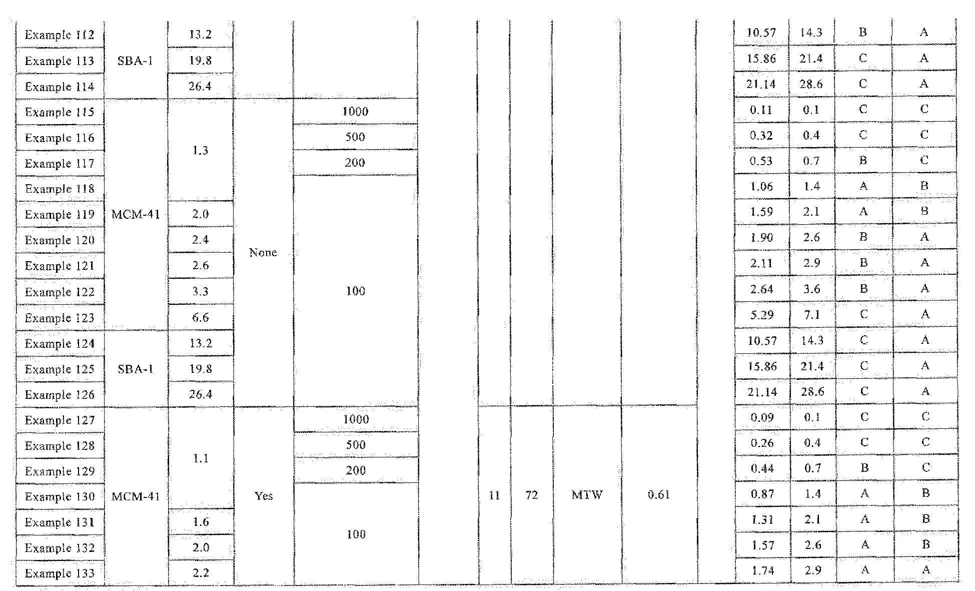

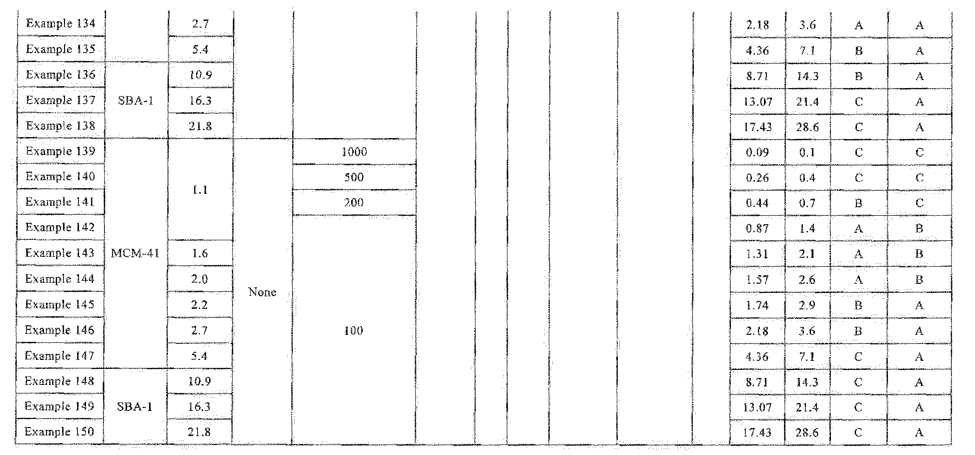

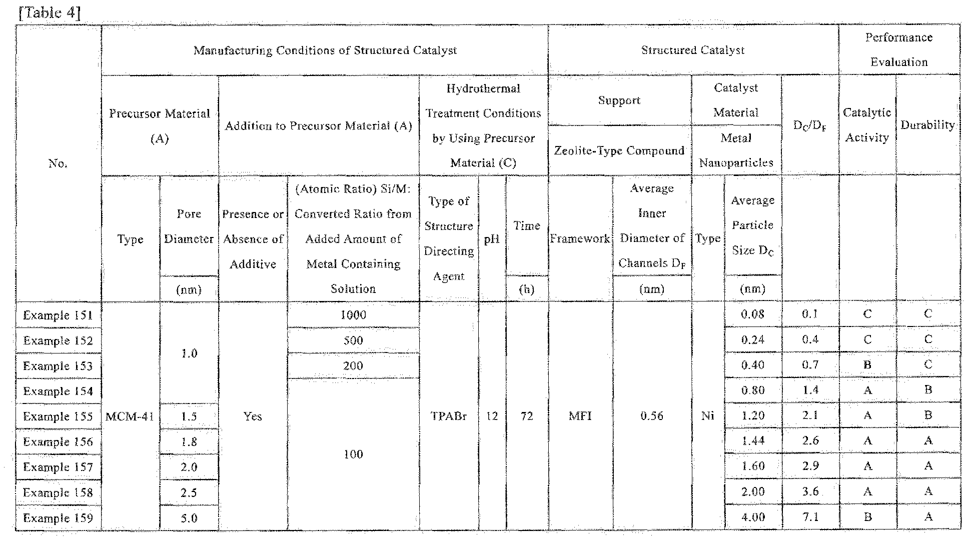

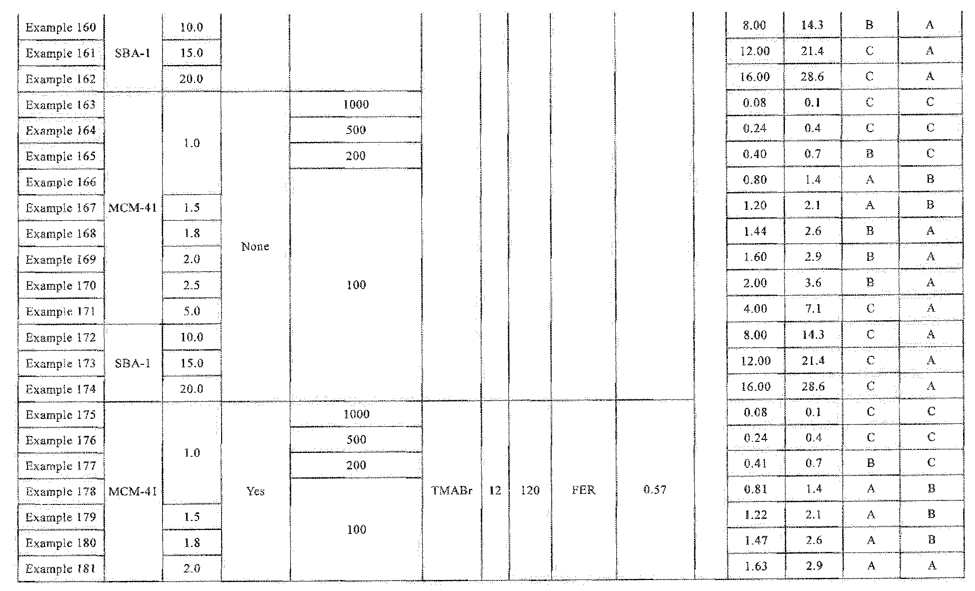

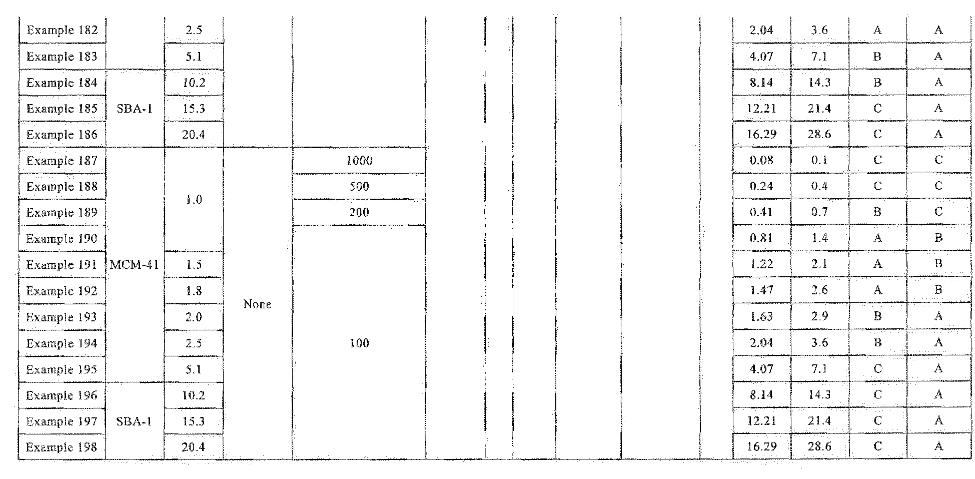

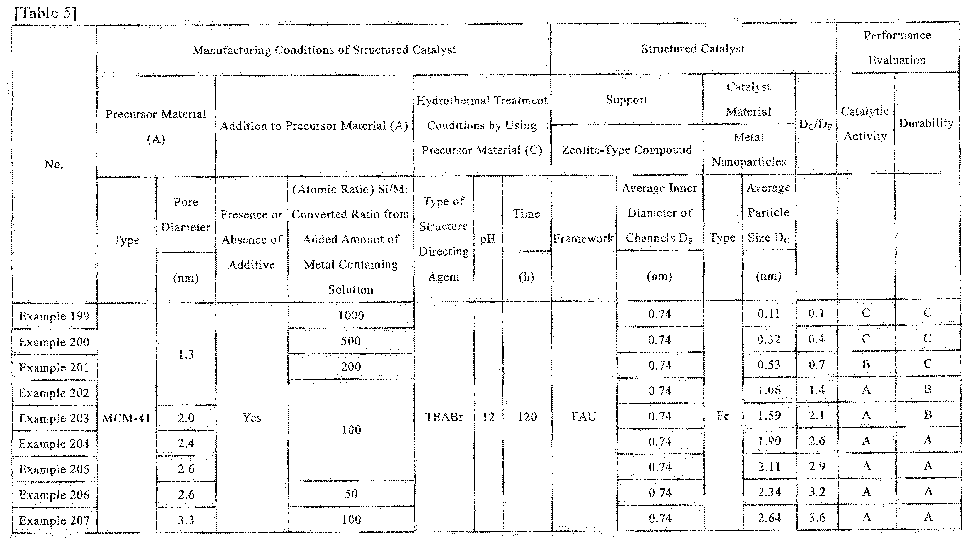

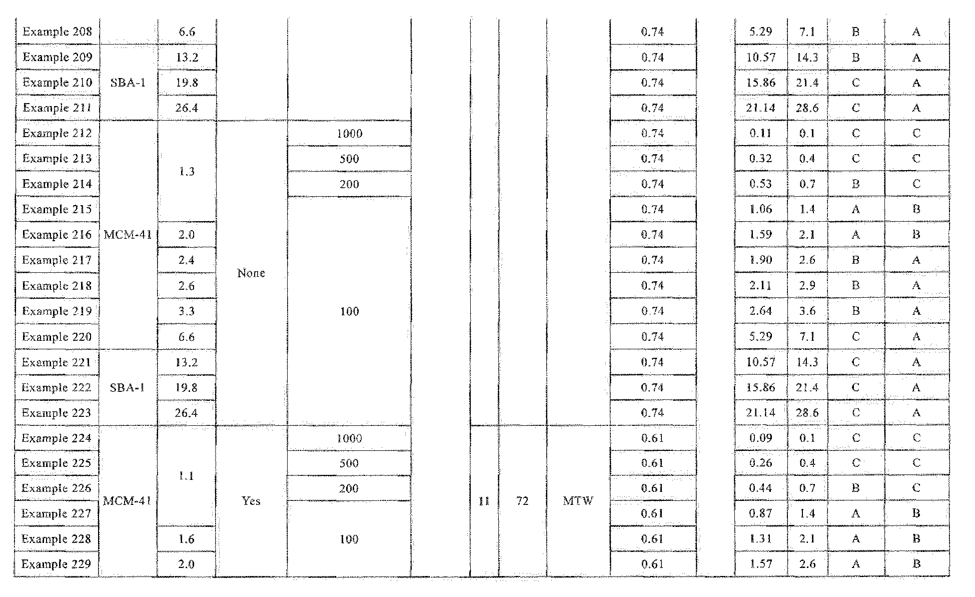

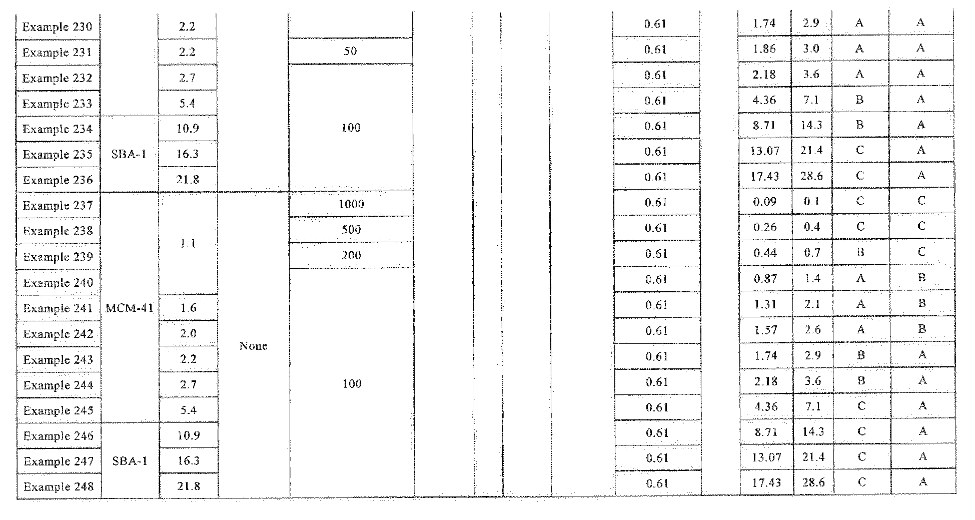

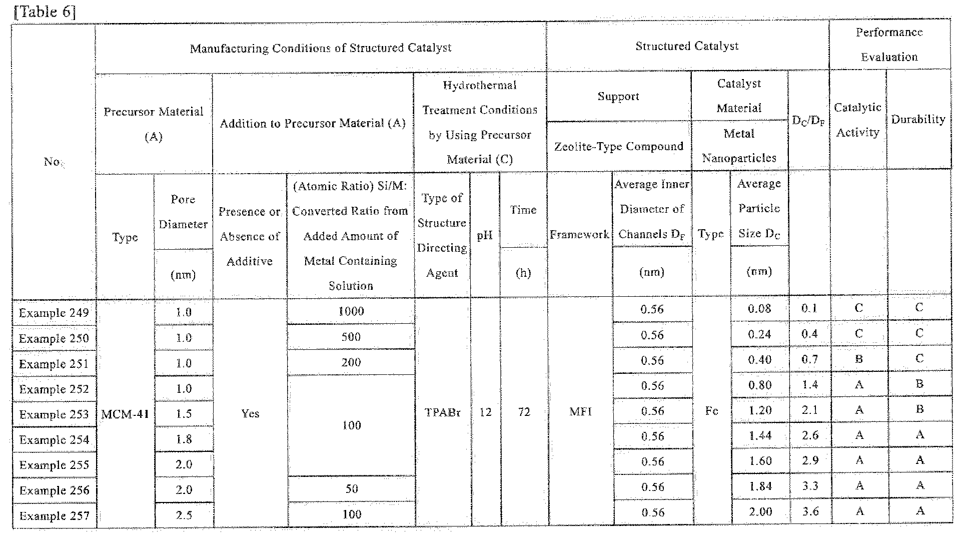

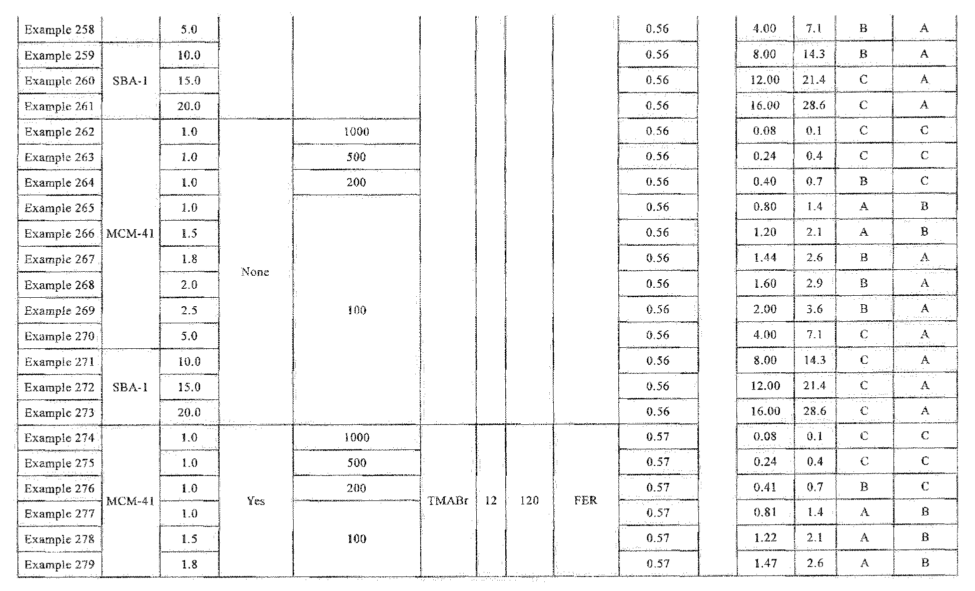

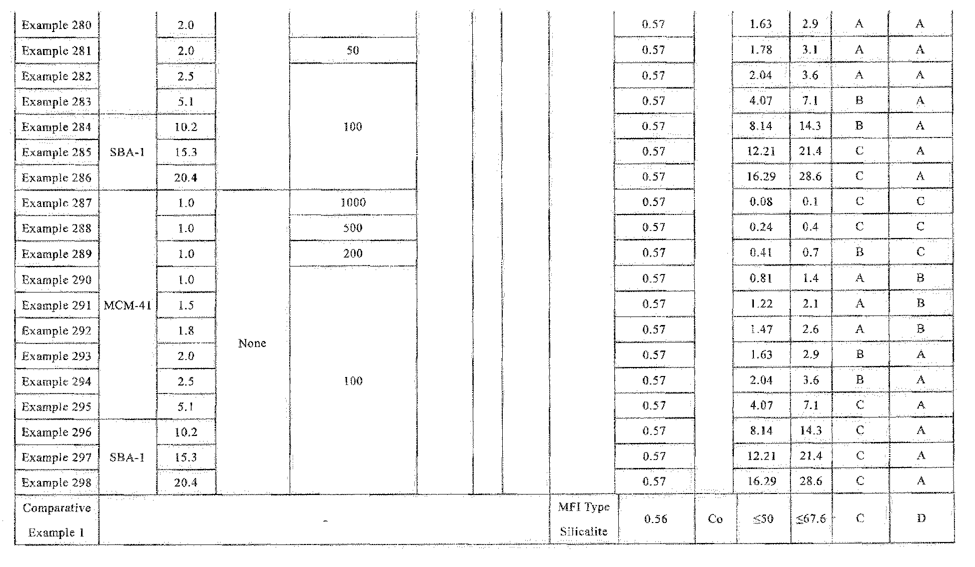

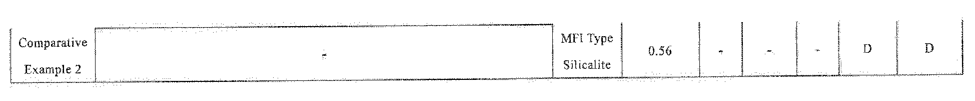

As it is clear from Tables 1 to 6, the catalyst structural bodies (Examples 1 to 298), which were confirmed by the cross-sectional observation that the catalyst materials were held within the support, were found to exhibit the excellent catalytic activity in the FT synthesis reaction and the excellent durability as a catalyst, compared to the structured catalyst in which the catalyst material was simply adhered to the outer surface of the support (Comparative Example 1), or the support itself without any catalyst material (Comparative Example 2).

In addition, a relationship between the amount of the metal (mass%) that was included inside the support of the structured catalyst measured in the evaluation [C] described above and the catalytic activity in the evaluation (1) described above was evaluated. The evaluation method was the same as the evaluation method performed in "(1) Catalytic Activity" in the [D] "Performance Evaluation" described above. As a result, in each example, when the added amount of the metal containing solution added to the precursor material (A) was adjusted to make the atomic ratio Si/M to be from 50 to 200 (the content of the metal element (M) of the metal nanoparticles with respect to the structured catalyst was from 0.5 to 2.5 mass%), it was found that the catalytic activity tended to improve in the FT synthesis reaction.

In addition, in order to compare the catalytic activities of the structured catalyst in which Co nanoparticles were included and the structured catalyst in which Fe nanoparticles were included, respective GC/MS areas (C₅ to C₂₀) of Examples 58 to 61 (structured catalyst including Co) and Examples 254 to 257 (structured catalyst including Fe) as typical examples in which both catalytic activity and durability were good, were compared. As a result, the structured catalyst including Co had a peak area of approximately 1.4 times that of the structured catalyst including Fe. From these results, it was found that Co is more suitable than Fe for the metal species to be included.

On the other hand, although the structured catalyst of Comparative Example 1, in which the catalyst material was adhered only to the outer surface of the support, exhibited better catalytic activity in the FT synthesis than the support itself of Comparative Example 2, which did not have any catalyst material, the structured catalyst of Comparative Example 1 exhibited poorer durability as a catalyst, compared to the catalyst structural bodies of Examples 1 to 298.

From the above results, it can be inferred that the catalyst structural bodies (Examples 1 to 298) exhibit the excellent catalytic activity even in the FT synthesis reaction in which the liquid hydrocarbon is synthesized from carbon monoxide and hydrogen, and that the catalyst structural bodies have the excellent durability as a catalyst.

### Other Embodiment

A method for using a structured catalyst for Fischer-Tropsch synthesis, in which the structured catalyst includes
a support of a porous structure including a zeolite-type compound and one or more metal nanoparticles present in the support,
the support including channels connecting with each other, and
the metal nanoparticles being present at least at an enlarged pore portion of the channel of the support.

### Reference Signs List

- 1: structured catalyst for Fischer-Tropsch synthesis
- 10: Support
- 10a: Outer surface
- 11: Channel
- 11a: Pore
- 12: Enlarged pore portion
- 20: Metal nanoparticles
- 30: Metal nanoparticles
- D_{C}: Average particle size
- D_{F}: Average inner diameter
- D_{E}: Inner diameter

## Claims

1. A structured catalyst for Fischer-Tropsch synthesis, comprising:
a support of a porous structure including a zeolite-type compound; and
one or more metal nanoparticles present in the support, wherein
the support has channels connecting with each other, and
the metal nanoparticles is present at least at the channel of the support.

2. The structured catalyst for Fischer-Tropsch synthesis according to claim 1, wherein
the channels have any one of a one-dimensional pore, a two-dimensional pore, and a three-dimensional pore defined by a framework of the zeolite-type compound and an enlarged pore portion different from any of the one-dimensional pore, the two-dimensional pore, and the three-dimensional pore, and
the metal nanoparticles are present at least in the enlarged pore portion.

3. The structured catalyst for Fischer-Tropsch synthesis according to claim 2, wherein
the enlarged pore portion causes a plurality of pores constituting any one of the one-dimensional pore, the two-dimensional pore, and the three-dimensional pore to connect with each other.

4. The structured catalyst for Fischer-Tropsch synthesis according to claim 2 or 3, wherein
an average particle size of the metal nanoparticles is larger than an average inner diameter of the channels and is smaller than or equal to an inner diameter of the enlarged pore portion.

5. The structured catalyst for Fischer-Tropsch synthesis according to any one of claims 1 to 4, wherein
a metal element (M) of the metal nanoparticles is included in an amount from 0.5 to 2.5 mass% with respect to the structured catalyst for Fischer-Tropsch synthesis.

6. The structured catalyst for Fischer-Tropsch synthesis according to any one of claims 1 to 5, wherein
the metal nanoparticles include one of Co, Fe, Ni, and an alloy containing at least one type of Co, Fe, or Ni.

7. The structured catalyst for Fischer-Tropsch synthesis according to any one of claims 1 to 6, wherein
the average particle size of the metal nanoparticles is from 0.08 to 30 nm.

8. The structured catalyst for Fischer-Tropsch synthesis according to claim 7, wherein
the average particle size of the metal nanoparticles is from 0.4 to 11.0 nm.

9. The structured catalyst for Fischer-Tropsch synthesis according to any one of claims 1 to 8, wherein
a ratio of the average particle size of the metal nanoparticles to the average inner diameter of the channels is from 0.05 to 300.

10. The structured catalyst for Fischer-Tropsch synthesis according to claim 9, wherein
the ratio of the average particle size of the metal nanoparticles to the average inner diameter of the channels is from 0.1 to 30.

11. The structured catalyst for Fischer-Tropsch synthesis according to claim 10, wherein
the ratio of the average particle size of the metal nanoparticles to the average inner diameter of the channels is from 1.4 to 3.6.

12. The structured catalyst for Fischer-Tropsch synthesis according to any one of claims 1 to 11, wherein
the channels include any one of a one-dimensional pore, a two-dimensional pore, and a three-dimensional pore defined by the framework of the zeolite-type compound and an enlarged pore portion different from any of the one-dimensional pore, the two-dimensional pore, and the three-dimensional pore,
the average inner diameter of the channels is from 0.1 to 1.5 nm, and the inner diameter of the enlarged pore portion is from 0.5 to 50 nm.

13. The structured catalyst for Fischer-Tropsch synthesis according to any one of claims 1 to 12, further comprising:
one or more other metal nanoparticles held on an outer surface of the support.

14. The structured catalyst for Fischer-Tropsch synthesis according to claim 13, wherein
a content of the one or more metal nanoparticles present in the support is larger than a content of the one or more other metal nanoparticles held on the outer surface of the support.

15. The structured catalyst for Fischer-Tropsch synthesis according to any one of claims 1 to 14, wherein
the zeolite-type compound is a silicate compound.

16. A manufacturing apparatus for hydrocarbon comprising the structured catalyst for Fischer-Tropsch synthesis according to any one of claims 1 to 15.

17. A method for manufacturing a structured catalyst for Fischer-Tropsch synthesis, comprising:
calcinating a precursor material (B) obtained by impregnating a precursor material (A) for obtaining a support having a porous structure composed of zeolite-type compound with a metal containing solution;
performing a hydrothermal treatment on a precursor (C) obtained by the calcinating the precursor material (B); and
reducing the hydrothermal-treated precursor material (C).

18. The method for manufacturing a structured catalyst for Fischer-Tropsch synthesis according to claim 17, wherein
from 50 to 500 mass% of a non-ionic surfactant is added with respect to the precursor material (A) before the calcinating.

19. The method for manufacturing a structured catalyst for Fischer-Tropsch synthesis according to one of claims 17 and 18, wherein
the impregnating the precursor material (A) with the metal containing solution is performed by adding the metal containing solution to the precursor material (A) in multiple portions prior to the calcinating.

20. The method for manufacturing a structured catalyst for Fischer-Tropsch synthesis according to any one of claims 17 to 19, wherein
in the impregnating the precursor material (A) with the metal containing solution before the calcinating, an added amount of the metal containing solution added to the precursor material (A) is adjusted to make a ratio of silicon (Si) constituting the precursor material (A) to a metal element (M) included in the metal containing solution added to the precursor material (A) (atomic ratio Si/M) to be from 10 to 1000.

21. The method for manufacturing a structured catalyst for Fischer-Tropsch synthesis according to claim 17, wherein
in the performing the hydrothermal treatment, the precursor material (C) and a structure directing agent are mixed.

22. The method for manufacturing a structured catalyst for Fischer-Tropsch synthesis according to claim 17, wherein
the performing the hydrothermal treatment is performed under a basic condition.

23. A method for manufacturing a liquid hydrocarbon by synthesizing the liquid hydrocarbon from carbon monoxide and hydrogen by using a catalyst, the catalyst comprising:
a support of a porous structure including a zeolite-type compound; and
one or more metal nanoparticles present in the support,
a structured catalyst for Fischer-Tropsch synthesis including the support with channels connecting with each other, and
the metal nanoparticles being present at least at an enlarged pore portion of the channel of the support.
